(19) Europäisches Patentamt
European Patent Office
Office européen des brevets

(11) Veröffentlichungsnummer: **0 268 014**
**A2**

(12) # EUROPÄISCHE PATENTANMELDUNG

(21) Anmeldenummer: **87108469.5**

(22) Anmeldetag: **11.06.87**

(51) Int. Cl.[4]: **C12N 15/00**, //A61K39/245,G01N33/569

Die Bezeichnung der Erfindung wurde geändert (Richtlinien für die Prüfung im EPA, A-III, 7.3).

Der (Die) Mikroorganismus (Mikroorganismen) ist (sind) bei der National Collection of Industrial Bacteria unter der (den) Nummer(n) NCIB 11 669; NCIB 12 163; NCIB 12 319 hinterlegt worden.

(30) Priorität: **13.06.86 DE 3619902**

(43) Veröffentlichungstag der Anmeldung:
**25.05.88 Patentblatt 88/21**

(84) Benannte Vertragsstaaten:
**BE CH DE FR GB LI NL**

(71) Anmelder: **Gesellschaft für Biotechnologische Forschung mbH (GBF)**
**Mascheroder Weg 1**
**D-3300 Braunschweig-Stöckheim(DE)**

(72) Erfinder: **Collins, John, Dr.**
**Ägidienweg 5**
**D-3300 Braunschweig(DE)**
Erfinder: **Lindenmaier, Werner, Dr.**
**Treppenkamp 10**
**D-3320 Salzgitter(DE)**
Erfinder: **Bonewald, Renate**
**Schützenstrasse 15**
**D-3300 Braunschweig(DE)**
Erfinder: **Necker, Antje**
**Jasperallee 66**
**D-3300 Braunschweig(DE)**

(74) Vertreter: **Boeters, Hans Dietrich, Dr. et al**
**Boeters, Bauer & Partner**
**Thomas-Wimmer-Ring 14**
**D-8000 München 22(DE)**

(54) **Cytomegalovirus-DNA-Strukturen, Expressionsvektor dafür, CMV-Protein-Strukturen und deren Verwendung.**

(57) Die Erfindung betrifft aus Cytomegalie-Virus (CMV) gewinnbare DNA-Strukturen, Expressionsvektoren für die DNA-Strukturen, von den DNA-Strukturen kodierte Proteinstrukturen und deren Verwendung.

EP 0 268 014 A2

Xerox Copy Centre

## CMV-DNA-Strukturen, Expressionsvektor dafür, CMV-Proteinstrukturen und deren Verwendung

Das Cytomegalievirus (CMV)[1-5] ist ein humanpathogenes Virus, das sehr weit verbreitet ist, aber im allgemeinen nur harmlose Krankheitserscheinungen verursacht. In Mitteleuropa haben etwa 50 % der 18-bis 30-jährigen eine CMV-Infektion durchgemacht und sind daher seropositiv. CMV kann latent in Zellen weiter (lebenslang?) vorhanden sein und unter Umständen reaktiviert werden. In Ländern mit weniger hygienischen Verhältnissen sind bis zu 100 % seropositiv[1]. Wesentliche pathogene Effekte hat eine Infektion dann, wenn das Immunsystem des Betroffenen nicht voll funktionsfähig ist. Prä-und perinatale Infektionen können zu schweren Infektionen führen und Hirnschäden unterschiedlichen Ausmaßes verursachen[2,3,5]. CMV wird für einen relativ großen Anteil der frühkindlichen geistigen Behinderungen verantwortlich gemacht. Bei Erwachsenen (und größeren Kindern) sind vor allem immunsupprimierte Patienten be troffen (Organtransplantation, Krebstherapie, evtl. Aids)[4]. CMV Infektion sind in solchen Fällen relativ oft lethal.

Es gibt bisher keine Therapie und keine Impfung. Vorbeugend kann z.B. bei Organtransplantationen passive Immunisierung mit Hyperimmunserum durchgeführt werden. Um Infektionen in seronegativen Empfängern zu vermeiden, ist die Verwendung von seronegativen Blutspenden wichtig[3,4].

Um den Immunstatus insbesondere von Transplantationspatienten, Schwangeren und Blutspendern zu kennen, ist gegebenenfalls eine geeignete schnelle Diagnostik notwendig. Ein besseres Verständis der antigenen Struktur des CMV und der CMV-infizierten Zellen wird voraussichtlich auch zur Entwicklung eines ungefährlichen CMV-Impfstoffes führen können; man vergleiche die Diskussion bei Herpes[16,17].

Es gibt prinzipiell zwei Methoden der CMV-Diagnostik[5,11,15].

1) Virus-Nachweis
2) Nachweis von Antikörpern gegen CMV

Zu 1) gibt es mindestens drei Varianten. Dazu zählt Kultivierung des CMV, Hybridisierung mit DNA-Proben und Nachweis mit (monoklonalen) Antikörpern. Als Ergebnis erhält man Auskunft über das Vorliegen einer aktuellen CMV-Infektion. Kultivierung ist sensitiv, aber aufwendig und langwierig, da Zellkulturen notwendig sind und CMV nur auf humanen primären Zellen wächst. Die anderen Methoden sind in einem eher experimentellen Stadium.

Zu 2) gibt es eine Vielzahl von technischen Varianten, die sich in Sensitivität und Leichtigkeit der Handhabung wesentlich unterscheiden. Das Prinzip ist jedoch immer gleich: Im Serum von Patienten werden Antikörper nachgewiesen, die vorgefertigte markierte (markierbare) CMV-Antigene binden. Man erhält Auskunft über den Immunstatus des Patienten. Eine Differenzierung des Antikörpertyps (IgG/IgM) und Verfolgung des zeitlichen Verlaufs erlaubt u.a. Aussagen über Neuinfektionen.

Diese Tests sind zum Teil relativ weit entwickelt und standardisiert [11]. Die Schwierigkeit liegt in der aufwendigen und wohl nicht immer reproduzierbaren Herstellung des CMV-Antigens (gereinigte Viren oder (meist) Extrakte aus infizierten Zellen). Dazu muß wieder CMV in Zellkultur kultiviert werden.

Die Verwendung von reproduzierbaren und billig in E. coli hergestellten CMV-Antigenen wird hier eine wesentliche Verbesserung bringen.

Aufgabe der Erfindung ist es, aus CMV gewinnbare DNA-Strukturen, Expressionsvektoren mit diesen DNA-Strukturen, von diesen DNA-Strukturen kodierte Proteinstrukturen und die Verwendung dieser Proteinstrukturen zum Nachweis von menschlichen Antikörpern gegen CMV-Proteine oder CMV vorzusehen. Es ist erwünscht, antigene Determinanten von CMV in E. coli zu produzieren, damit diese antigenen Determinanten von Anti-CMV-Antikörpern der Patienten erkannt werden, so daß das aus infizierten Zellen gewonnene Antigen für diagnostische Zwecke ersetzt werden kann. Die Produktion sollte billiger und reproduzierbarer sein. Damit ergäbe sich eine Erweiterung und Verbesserung der diagnostischen Möglichkeiten. Zur genannten Aufgabe gehört es auch, aus Klonen gewonnene Fusionsproteine direkt (allein oder in Kombination) oder modifizierte (beispielsweise durch Protease abgespaltene) Peptide als Antigene einzusetzen.

Die der Erfindung zugrundeliegende Aufgabe wird durch die Gegenstände der Ansprüche gelöst.

Nachstehend wird die Erfindung näher erläutert.

### Experimenteller Teil

Die Klonierung von offenen Leserahmen (ORF = open reading frame), das heißt Genabschnitten, die potentiell für ein Protein kodieren könnten, ist eine sehr effektive Genisolierungsmethode, wenn ein immunologischer Nachweis des Peptids möglich ist. Es ist üblich, ORFs als Fusionsproteine zu exprimieren.

Regulationssignale für Transcription und Translation stammen von Genen aus E. coli oder E.-coli-Bakteriophagen. Der ORF wird z.B. derart mit einem E.-coli-Gen (meist β-Galaktosidasegen) gekoppelt, daß das Produkt ein Fusionsprotein darstellt. Das erhöht die Expression und die Stabilität des Peptids. Die Methode ist zum Nachweis sowohl von cDNA-Bereichen als auch von Exons genomischer DNA verwendbar.

ORF-Klonierungsvektoren gibt es sowohl als Derivate von lambda-Phagen (lambda gt 11)[6,7] als auch auf der Basis von verschiedenen Plasmiden[8,9]. Es lassen sich verschiedene Klonierungsstellen und Promotoren (lac, tac, $P_1$) verwenden. Die Fusion kann entweder am N-Terminus oder am Carboxy-Terminus der β-Galaktosidase erfolgen[8,9]. Bei Anbau am C-Terminus ist die statistische Wahrscheinlichkeit für richtige Orientierung und richtigen Leserahmen der Sequenz 1/6. Bei Inserion am N-Terminus beträgt sie 1/18, jedoch ist dann die Aktivität der β-Galaktosidase in der Regel erhalten, so daß das Fusionsprotein gleich mit einem enzymatischen Marker versehen ist[8].

Der immunologische Nachweis spezifischer Peptidsequenzen erfolgt nach Übertragung der Fusionsproteine produzierenden E.-coli-Kolonien auf Nitrocellulosefilter. Nach Lysis der Zellen binden die Proteine an das Filter[13]. Inkubation mit spezifischem Antiserum und Nachweis der spezifischen Bindung mit z.B. zweiten markierten Antikörpern erlaubt die Identifizierung der positiven Kolonie, die dazu von der "Master"-Platte gepickt werden kann[12,13].

1) Klonierung von antigenen Determinanten von CMV

a) Vektoren

Für die erfindungsgemäßen Arbeiten wurden als ORF-Expressionsvektoren pEX-Vektoren benutzt, und zwar (1) pEX2[9] und (2) eine Variante von pEX1 (pEXStu). Für pEXStu wurde ein synthetischer DNA-Linker eingebaut, der vor der StuI-Schnittstelle Sequenzen für ein Peptid enthält, das von einer Protease (Faktor Xa) spezifisch geschnitten werden kann. Diese Konstruktion macht es möglich, die CMV-Peptide mit der spezifischen Protease (Faktor Xa) vom Rest des Fusionsproteins abzuspalten[10]. Die glatte StuI-Schnittstelle dient zum Einbau der Insert-CMV-DNA.

pEX-Vektoren benutzen den PL-Promotor zur Expression des Fusionsproteins. Dieser wird durch den lambda-Repressor (cI) eines koexistierenden Plasmids kontrolliert. Durch Verwendung eines temperatursensitiven Repressors (cI ts) kann die Expression durch Temperaturerhöhung eingeschaltet werden[9]. Erfindungsgemäß wurden E.-coli-Stämme (5K, DH1) benutzt, die diesen Repressor auf dem Plasmid pRK248cIts 857 enthalten. Es wurden zwei ORF-Genbanken hergestellt, die eine mit pEX2 mit der "tailing"-Methode (mit PstI geschnittener Vektor mit Oligo-dG-Schwänzen), die andere durch Klonierung von Blunt-end-Fragmenten in die StuI-Stelle von pEXStu.

b) Insert DNA

Als Insert-DNA für die CMV-ORF-Genbanken wurde klonierte DNA benutzt, und zwar Klone der Cosmidbank von CMV Ad169[18], die das gesamte CMV-Genom abdecken. Durch Behandlung mit DNAseI in Gegenwart von $Mn^{2+}$ wurden die DNA fragmentiert und Fragmente von ca. 300 bis 600 bp isoliert. Für die erste Genbank wurden Oligo-C-Schwänze mit terminaler Transferase angehängt. Diese Fragmente wurden an die komplementären Oligo-G-Schwänze des pEX2-Vektors hybridisiert. Für die Blunt-end-Klonierung in der StuI-Schnittstelle von pEXStu wurden die Fragmente mit T4-Polymerase aufgefüllt.

c) Genbanken

Genbank 1: pEX2-CMV, kloniert nach der Oligo-G/C-Methode. Es wurden 34 000 unabhängige Kolonien isoliert. Ca. 50 % hatten größere Inserts (ca. 300 bp).

Genbank 2: pEXStu-CMV. Blunt-end-Klonierung in der StuI-Stelle. Von $3.2 \times 10^5$ unabhängigen Kolonien hatten wiederum 50 % Inserts von ca. 300 bp.

Als Wirtsstämme wurde E. coli 5K (pRK248cIts) bzw. DH1 (pRK248cIts) verwendet. Die Kolonien wurden resuspendiert und bei - 70 °C eingefroren.

d) Immunscreening

E.-coli-Klone wurden auf Nitrozellulosefilter übertragen und die Produktion des Fusionsproteins induziert. Nach Lyse der Zellen wurden unspezifische Bindungsstellen abgesättigt (Biorad-Methode)[12] und mit einem anti-CMV-Serum (Polyscience) inkubiert. Spezifische Bindung der Antikörper wurde mit Schwein/anti-Ziege-Ig-Serum nachgewiesen. Färbung erfolgte mit Chloronaphtol/$H_2O_2$.

e) Immunologische Charakterisierung

Beim Screening positive Kolonien wurden weiter untersucht. Die Proteine wurden extrahiert und in einem SDS-Polyacrylamidgel aufgetrennt. Die Antigenität des Fusionsproteins wurde nach Übertragung auf Nitrocellulosefilter (Westernblot)[14] untersucht. Es wurden Ziege-anti-CMV-Serum (Polysciene), human-anti-CMV-Serum (gemischtes Hyperimmunserum von Biotest), Kaninchen-anti-CMV-Serum (Fleckenstein, Erlangen) und Kontrollseren getestet. Bei einigen Klonen ergab sich eine starke positive Reaktion mit Human-Serum, auch die meisten individuellen CMV-positiven Seren reagierten.

f) Sequenzierung

Die Sequenzen und der Leserahmen der positiven Klone wurden bestimmt. Die Klone der Genbank 1 wurde nach der Maxam-Gilbert-Methode sequenziert, Klone der Ganbank 2 mit der Dideoxy-Methode im M13. Die ermittelten Sequenzen sind den Ansprüche 1 bis 4 zu entnehmen.

Literaturverzeichnis

1) St.A. Plotkin, Immunology of Cytomegalovirus, Immunology of Human Infection 9, Comprehensive Immunology, S. 89 -106, Plenum Publishing Corp., New York 1982
2) Science, 190 (1975) 1184 - 1186
3) Th. Luthardt, Klinische Bedeutung der Verabreichung von CMV-seronegativen Blutes bei Früh-und Neugeborenen, Transfusionsbedingte Zytomegalievirusinfektionen, S. 1 - 5, Steinkopff-Verlag, Darmstadt 1984
4) H. Bunzendahl, Erkrankung durch Zytomegalievirus nach Organtransplantation, Transfusionsbedingte Zytomegalievirusinfektionen, S. 15 - 19, Steinkopff-Verlag, Darmstadt 1984
5) Medizin in unserer Zeit, 2 (1978) 185 - 193
6) Proc. Natl. Acad. USA, 82 (1985) 1266 - 1270
7) Proc. Natl. Acad. USA, 80 (1983) 1194 - 1198
8) Proc. Natl. Acad. USA, 79 (1982) 6852 - 6855
9) The Embo Journal, 3 (1984) 1429 - 1434
10) Nature, 309 (1984) 810 - 812
11) Enzym-Immuno-Assay zur Bestimmung von Cytomegalie-Virus-(CMV)-IgM-Antikörpern, medac Gesellschaft für klinische Spezialpräparate mbH, Prospekt 1983
12) Express-Blot™ Assay Kit, Instruction Manual, Bio-Rad Laboratories, Richmond, USA, 1985
13) Nucleic Acids Research, 13 (1985) No. 13, 19 S., C.L. Verweij et al., Construction of cDNA coding for human von Willebrand factor etc.
14) Anal. Biochem., 112 (1981) 195 - 203
15) Middeldorp, Thesis, Rijksuniversiteit Groningen 1985
16) Nature, 302 (1983) 72 - 74
17) Biotechnology (1984) 527 - 532
18) Gene, 18 (1982) 39 - 46

**Ansprüche**

1. Aus Cytomegalie-Virus (CMV) gewinnbare DNA-Struktur der folgenden Basensequenz (CMV-1; wobei zusätzlich die dazu korrespondierende Aminosäuresequenz angegeben ist):

GlnGlnGlnArgHisAlaAlaPheSerLeuValSerProGlnValThrLysAlaSerPro
CAACAACAGCGTCACGCGGCTTTCAGTCTCGTCTCCCCGCAGGTGACCAAGGCCAGCCCG
GTTGTTGTCGCAGTGCGCCGAAAGTCAGAGCAGAGGGGCGTCCACTGGTTCCGGTCGGGC

GlyArgValArgArgAspSerAlaTrpAspValArgProLeuThrGluThrArgGlyAsp
GGAAGGGTCCGTCGGGACAGCGCGTGGGACGTGAGGCCGCTCACGGAGACCAGAGGGGAT
CCTTCCCAGGCAGCCCTGTCGCGCACCCTGCACTCCGGCGAGTGCCTCTGGTCTCCCCTA

LeuPheSerGlyAspGluAspSerAspSerSerAspGlyTyrProProAsnArg
CTTTTCTCGGGCGACGAGGATTCCGACAGCTCGGATGGCTATCCCCCCAACCGT
GAAAAGAGCCCGCTGCTCCTAAGGCTGTCGAGCCTACCGATAGGGGGGTTGGCA

2. Aus Cytomegalie-Virus (CMV) gewinnbare DNA-Struktur der folgenden Basensequenz (CMV-2; wobei zusätzlich die dazu korrespondierende Aminosäuresequenz angegeben ist):

AlaProGluArgCysSerGlySerleuSerValSerThrLeuLeuArgGlnHisProSer
GCCCCTGAAAGATGCTCTGGGTCGCCAAGTGTCTCTACGCTCCTACGACAACATCCCTCC
CGGGGACTTTCTACGAGACCCAGCGGTTCACAGAGATGCGAGGATGCTGTTGTAGGGAGG

AspPheLeuLeuGlyArgGlyGluAspAspAspAspGlyGluAspAspValThrArgSer
GACTTCCTCCTCGGACGAGGGGAGGACGATGACGACGGGGAGGATGACGTAACGAGGAGC
CTGAAGGAGGAGCCTGCTCCCCTCCTGCTACTGCTGCCCCTCCTACTGCATTGCTCCTCG

3. Aus Cytomegalie-Virus (CMV) gewinnbare DNA-Struktur der folgenden Basensequenz (CMV-13; wobei zusätzlich die dazu korrespondierende Aminosäuresequenz angegeben ist):

LeuThrProThrProValAsnProSerThrAlaProAlaProAlaProThrProThrPhe
CTCACGCCGACGCCTGTCAATCCTTCCACGGCCCCCGCTCCGGCCCCGACACCTACCTTC
GAGTGCGGCTGCGGACAGTTAGGAAGGTGCCGGGGGCGAGGCCGGGGCTGTGGATGGAAG

AlaGlyThrGlnThrProValAsnGlyAsnSerProTrpAlaProThrAlaProLeuPro
GCGGGTACCCAAACCCCGGTCAACGGTAACTCGCCCTGGGCTCCGACGGCGCCGTTGCCC
CGCCCATGGGTTTGGGGCCAGTTGCCATTGAGCGGGACCCGAGGCTGCCGCGGCAACGGG

GlyAspMetAsnProAlaAsnTrpProArgGluArgAlaTrpAlaLeuLysAsnProHis
GGGGATATGAACCCCGCCAACTGGCCGCGCGAACGCGCGTGGGCCCTCAAGAATCCTCAC
CCCCTATACTTGGGGCGGTTGACCGGCGCGCTTGCGCGCACCCGGGAGTTCTTAGGAGTG

LeuAlaTyrAsnProPheArgMetProThr
CTGGCTTACAATCCCTTCAGGATGCCTACG
GACCGAATGTTAGGGAAGTCCTACGGATGC

4. Aus Cytomegalie-Virus (CMV) gewinnbare DNA-Struktur der folgenden Basensequenz (CMV-21; wobei zusätzlich die dazu korrespondierende Aminosäuresequenz angegeben ist):

AspGluGluAspThrSerIleTyrLeuSerProProProValProProValGlnValVal
GACGAAGAGGACACCTCAATTTACCTGTCCCCGCCCCCGGTCCCCCCCGTCCAGGTGGTG
CTGCTTCTCCTGTGGAGTTAAATGGACAGGGGCGGGGGCCAGGGGGGGCAGGTCCACCAC

AlaLysArgLeuProArgProAspThrProArgThrProArgGlnLysLysIleSerGln
GCTAAGCGACTGCCGCGGCCCGACACACCCAGGACTCCGCGCCAAAAGAAGATTTCACAA
CGATTCGCTGACGGCGCCGGGCTGTGTGGGTCCTGAGGCGCGGTTTTCTTCTAAAGTGTT

ArgProProThrProGlyAsp
CGTCCACCCACCCCCGGGATCC
GCAGGTGGGTGGGGGCCCCTAGG

5. Aus Cytomegalie-Virus (CMV) gewinnbare DNA-Struktur der folgenden Basensequenz (CMV-38; wobei zusätzlich die dazu korrespondierenden Aminosäuresequenzen aller 3 Leserahmen angegeben sind):

```
  LeuArgArgGlyThrProGlyGlnSerGlnLysGlnHisArgHisGlyGlySerGly
 ArgTyrAspValValHisLeuValSerArgArgSerSerThrValThrGlyAlaAlaAla
  ValThrThrTrpTyrThrTrpSerValAlaGluAlaAlaProSerArgGlyGlnArgArg
 CGTTACGACGTGGTACACCTGGTCAGTCGCAGAAGCAGCACCGTCACGGGGGCAGCGGCG
 GCAATGCTGCACCATGTGGACCAGTCAGCGTCTTCGTCGTGGCAGTGCCCCCGTCGCCGC

GlyHisAsnLysArgArg***GlyLysProArgArgArgArgArgArgProArgThrLys
 AspThrThrAsnAlaGlyLysGlyAsnArgGlyGlyValValValValLeuGlyArgArg
  ThrGlnGlnThrProValArgGluThrAlaAlaAlaSerSerSerSerSerAspGluAsp
 GACACAACAAACGCCGGTAAGGAAACCGCGGCGGCGTCGTCGTCGTCCTCGGACGAAGA
 CTGTGTTGTTTGCGGCCATTCCCTTTGGCGCCGCCGCAGCAGCAGCAGGAGCCTGCTTCT

Thr***ValSerGlnAlaGlyArgAlaArgProGlyThrLysAlaSerLysLysSerArg
 LeuGluPheProArgGlnAlaGluHisGlyArgAlaArgLysArgLeuLysSerHisVal
  LeuSerPheProGlyArgProSerThrAlaGlyHisGluSerVal***LysValThrSer
 CTTGAGTTTCCCAGGCAGGCCGAGCACGGCCGGGCACGAAAGCGTCTAAAAAGTCACGTC
 GAACTCAAAGGGTCCGTCCGGCTCGTGCCGGCCCGTGCTTTCGCAGATTTTTCAGTGCAG

Gln***ArgArgTrpLysTrpArgAlaArgArgValProIleSerSerSerAsnAsnVal
 AsnSerAspGlyGlySerGlyGlyHisGlyGlyPheGlnSerAlaAlaAlaThrThrLeu
  IleAlaThrValGluValAlaGlyThrAlaGlySerAsnGlnGlnGlnGlnGlnArgTyr
 AATAGCGACGGTGGAAGTGGCGGCACGGCGGGTTCCAATCAGCAGCAGCAACAACGTTA
 TTATCGCTGCCACCTTCACCGCCCGTGCCGCCCAAGGTTAGTCGTCGTCGTTGTTGCAAT

Thr
 Arg

 CGA
 GCT  wobei *** ein Stopcodon bedeutet.
```

6. Aus Cytomegalie-Virus (CMV) gewinnbare DNA-Struktur der folgenden Basensequenz (CMV-65; wobei zusätzlich die dazu korrespondierende Aminosäuresequenz angegeben ist):

```
  HisAspGluGlyAlaAlaGlnGlyAspAspAspValTrpThrSerGlySerAspSer
 GGCACGACGAGGGTGCCGCCCAGGGCGACGACGACGTCTGGACCAGCGGATCGGACTCCG
 CCGTGCTGCTCCCACGGCGGGTCCCGCTGCTGCTGCAGACCTGGTCGCCTAGCCTGAGGC

AspGluGluLeuValThrThrGluProLysThrProArgValThrGlyGlyGlyAlaMet
 ACGAAGAACTCGTAACCACCGAGCCCAAGACGCCCCGCGTCACCGGCGGCGGCGCCATGG
 TGCTTCTTGAGCATTGGTGGCTCGGGTTCTGCGGGGCGCAGTGGCCGCCGCCGCGGTACC

AlaGlyAlaSerThrSerAlaGlyArgLysArgLysSerAlaSerSerAlaThrAlaCys
 CGGGCGCCTCCACTTCCGCGGGCCGCAAACGCAAATCAGCATCCTCGGCGACGGCGTGCA
 GCCCGCGGAGGTGAAGGCGCCCGGCGTTTGCGTTTAGTCGTAGGAGCCGCTGCCGCACGT

ThrSerGlyValMetThrArgGlyArgLeuLysAlaGluSerThrValAlaProGluGlu
 CGTCGGGCGTTATGACACGCGGCCGCCTTAAGGCCGAGTCCACCGTCGCGCCCGAAGAGG
 GCAGCCCGCAATACTGTGCGCCGGCGGAATTCCGGCTCAGGTGGCAGCGCGGGCTTCTCC

AspThrAspGluAspSerAspAsnGlu
 ACACCGACGAGGATTCCGACAACGAAAT
 TGTGGCTGCTCCTAAGGCTGTTGCTTTA
```

7. Aus Cytomegalie-virus (CMV) gewinnbare DNA-Struktur der folgenden Basensequenz (CMV-71; wobei zusätzlich die dazu korrespondierende Aminosäuresequenz angegeben ist):

LeuArgAlaGlyLeuLeuProArgArgGlnValMetArgGlyAlaValSerGluPheLeu
CTGCGCGCCGGTCTCTTACCACGGAGGCAAGTCATGCGCGGCGCCGTCTCCGAGTTTCTC
GACGCGCGGCCAGAGAATGGTGCCTCCGTTCAGTACGCGCCGCGGCAGAGGCTCAAAGAG

ProGlnSerProGlyLeuProProThrGluGluGluGluGluGluGluGluAspAsp
CCGCAGTCCCCCGGATTACCACCCACCGAGGAAGAGGAGGAAGAAGAGGAAGAGGACGAC
GGCGTCAGGGGGCCTAATGGTGGGTGGCTCCTTCTCCTCCTTCTTCTCCTTCTCCTGCTG

GluAspAspLeuSerSerThrProThrProThrProLeuSerGluAlaMetPheAlaGly
GAAGATGACCTCTCCTCCACACCGACGCCGACCCCCCTGTCCGAAGCCATGTTTGCCGGC
CTTCTACTGGAGAGGAGGTGTGGCTGCGGCTGGGGGGACAGGCTTCGGTACAAACGGCCG

PheGluGluAlaSerGlyAspGluAspSerAspThrGlnAlaGlyLeuSerProAlaLeu
TTCGAGGAAGCCAGCGGCGACGAGGACTCGGACACCCAAGCCGGACTGTCCCCGGCACTG
AAGCTCCTTCGGTCGCCGCTGCTCCTGAGCCTGTGGGTTCGGCCTGACAGGGGCCGTGAC

IleLeuThrGlyGlnArgArgArgSerGlyAsnAsnGlyAlaLeuThrLeuValIlePro
ATCCTGACCGGACAAAGACGTCGAAGCGGTAACAACGGGGCTCTCACGCTCGTCATCCCC
TAGGACTGGCCTGTTTCTGCAGCTTCGCCATTGTTGCCCCGAGAGTGCGAGCAGTAGGGG

SerTrpHisValPheAlaSerLeuAspAsp
TCGTGGCACGTCTTTGCGAGCCTTGACGAC
AGCACCGTGCAGAAACGCTCGGAACTGCTG

8. DNA-Struktur, deren Einzelstränge mit denen einer DNA-Struktur gemäß einem der vorhergehenden Ansprüche hybridisierbar sind, vorzugsweise bei einer Tempeatur von mindestens 20 °C und insbesondere bei einer Konzentration von 1 M NaCl und einer Temperatur von mindestens 25 °C.

9. Expressionsvektor, umfassend eine DNA-Struktur gemäß einem der vorhergehenden Ansprüche zur Expression dieser DNA-Struktur in Escherichia coli.

10. Proteinstruktur der folgenden Aminosäuresequenz:

GlnGlnGlnArgHisAlaAlaPheSerLeuValSerProGlnValThrLysAlaSerPro

GlyArgValArgArgAspSerAlaTrpAspValArgProLeuThrGluThrArgGlyAsp

LeuPheSerGlyAspGluAspSerAspSerSerAspGlyTyrProProAsnArg

11. Proteinstruktur der folgenden Aminosäuresequenz:

AlaProGluArgCysSerGlySerProSerValSerThrLeuLeuArgGlnHisProSer

AspPheLeuLeuGlyArgGlyGluAspAspAspAspGlyGluAspAspValThrArgSer

12. Proteinstruktur der folgenden Aminosäuresequenz:

LeuThrProThrProValAsnProSerThrAlaProAlaProAlaProThrProThrPhe

AlaGlyThrGlnThrProValAsnGlyAsnSerProTrpAlaProThrAlaProLeuPro

GlyAspMetAsnProAlaAsnTrpProArgGluArgAlaTrpAlaLeuLysAsnProHis

LeuAlaTyrAsnProPheArgMetProThr

13. Proteinstruktur der folgenden Aminosäuresequenz:

AspGluGluAspThrSerIleTyrLeuSerProProProValProProValGlnValVal

AlaLysArgLeuProArgProAspThrProArgThrProArgGlnLysLysIleSerGln

ArgProProThrProGlyAsp

14. Proteinstrukturen der folgenden Aminosäuresequenzen:

LeuArgArgGlyThrProGlyGlnSerGlnLysGlnHisArgHisGlyGlySerGly

GlyHisAsnLysArgArg***GlyLysProArgArgArgArgArgArgProArgThrLys

Thr***ValSerGlnAlaGlyArgAlaArgProGlyThrLysAlaSerLysLysSerArg

Gln***ArgArgTrpLysTrpArgAlaArgArgValProIleSerSerSerAsnAsnVal

Thr /

und

ArgTyrAspValValHisLeuValSerArgArgSerSerThrValThrGlyAlaAlaAla

AspThrThrAsnAlaGlyLysGlyAsnArgGlyGlyValValValValLeuGlyArgArg

LeuGluPheProArgGlnAlaGluHisGlyArgAlaArgLysArgLeuLysSerHisVal

AsnSerAspGlyGlySerGlyGlyHisGlyGlyPheGlnSerAlaAlaAlaThrThrLeu

Arg und

ValThrThrTrpTyrThrTrpSerValAlaGluAlaAlaProSerArgGlyGlnArgArg
ThrGlnGlnThrProValArgGluThrAlaAlaAlaSerSerSerSerSerAspGluAsp
LeuSerPheProGlyArgProSerThrAlaGlyHisGluSerVal***LysValThrSer
IleAlaThrValGluValAlaGlyThrAlaGlySerAsnGlnGlnGlnGlnGlnArgTyr

wobei *** einem stop codon entspricht.

15. Proteinstruktur der folgenden Aminosäuresequenz:

HisAspGluGlyAlaAlaGlnGlyAspAspAspValTrpThrSerGlySerAspSer

AspGluGluLeuValThrThrGluProLysThrProArgValThrGlyGlyGlyAlaMet

AlaGlyAlaSerThrSerAlaGlyArgLysArgLysSerAlaSerSerAlaThrAlaCys

ThrSerGlyValMetThrArgGlyArgLeuLysAlaGluSerThrValAlaProGluGlu

AspThrAspGluAspSerAspAsnGlu

16. Proteinstruktur der folgenden Aminosäuresequenz:

LeuArgAlaGlyLeuLeuProArgArgGlnValMetArgGlyAlaValSerGluPheLeu

ProGlnSerProGlyLeuProProThrGluGluGluGluGluGluGluGluGluAspAsp

GluAspAspLeuSerSerThrProThrProThrProLeuSerGluAlaMetPheAlaGly

PheGluGluAlaSerGlyAspGluAspSerAspThrGlnAlaGlyLeuSerProAlaLeu

IleLeuThrGlyGlnArgArgArgSerGlyAsnAsnGlyAlaLeuThrLeuValIlePro

SerTrpHisValPheAlaSerLeuAspAsp

17. Verwendung einer Proteinstruktur gemäß einem der vorhergehenden Ansprüche zum Nachweis von menschlichen Antikörpern gegen CMV-Proteine oder CMV.

18. Verwendung einer Proteinstruktur gemäß einem der vorhergehenden Ansprüche als Bestandteil eines Impfstoffs gegen CMV.

19. Verwendung einer Proteinstruktur, die durch die DNA-Struktur gemäß Anspruch 8 kodiert wird, zum Nachweis von menschlichen Antikörpern gegen CMV-Proteine oder CMV.

20. Verwendung einer Proteinstruktur, die durch die DNA-Struktur gemäß Anspruch 8 kodiert wird als Bestandteil eines Impfstoffes gegen CMV.